# EUROPÄISCHE PATENTSCHRIFT

(11) **EP 4 482 829 B1**
(45) Veröffentlichungstag und Bekanntmachung des Hinweises auf die Patenterteilung: **21.01.2026**
(21) Anmeldenummer: 23706734.3
(22) Anmeldetag: 20.02.2023
(51) Int. Cl.: C07D 307/68

(54) **VERFAHREN ZUR OXIDATION VON HYDROXYMETHYLFURFURAL**
PROCESS FOR THE OXIDATION OF HYDROXYMETHYLFURFURAL
PROCÉDÉ D'OXYDATION D'HYDROXYMÉTHYLFURFURAL

(30) Priorität: 21.02.2022 DE 102022201794
(43) Veröffentlichungstag der Anmeldung: 01.01.2025
(73) Patentinhaber: Südzucker AG, 68165 Mannheim (DE)
(72) Erfinder: KUNZ, Sebastian, 67590 Monsheim (DE); KRÖNER, Christine, 67271 Kindenheim (DE); WEINMANN, Marco, 67547 Worms (DE); HAJI BEGLI, Alireza, 67305 Ramsen (DE)
(74) Vertreter: Schrell, Andreas
(86) Internationale Anmeldenummer: PCT/EP2023/054244
(87) Internationale Veröffentlichungsnummer: WO 2023/156668

(56) Entgegenhaltungen:
- JP-A- 2009 029 751
- KR-B1- 101 936 966
- US-B2- 9 199 957

## Beschreibung

Die vorliegende Erfindung betrifft Verfahren zur Herstellung von 2,5-Furandicarboxylsäure (FDCA) aus Hydroyxymethylfurfural (HMF) durch 2-stufige Oxidation des HMF sowie Verfahren zur Herstellung von Polyestern, Polyamiden oder Polyurethanen aus dem erhaltenen FDCA.

5-Hydroxymethylfurfural (HMF) ist ein multifunktionales Molekül mit einem aromatischen 5-Ring-System, einer Aldehyd- und einer Alkoholgruppe. Die vielen Funktionalitäten machen das Molekül zu einer vielfältig einsetzbaren Plattformchemikalie, die als Basis für eine große Vielzahl weiterer Verbindungen dienen kann. Zu den Verbindungen, die auf Basis von HMF hergestellt werden können, gehören zum einen Chemikalien, die heute bereits im industriellen Maßstab auf petrochemischem Wege hergestellt werden, beispielsweise Caprolactam oder Adipinsäure, aber auch Verbindungen mit einem großen Anwendungspotential, für die bislang noch kein technischer Herstellprozess mit ausreichender Effizienz zur Verfügung steht, wie 2,5-Furandicarbonsäure (FDCA). FDCA kann als Monomer für die Herstellung von Polyethylenfuranoat (PEF) dienen, welches als nachhaltige Rohstoffbasis mit vorteilhaften technologischen Eigenschaften, wie einer verbesserten Gasbarrierewirkung, in Konkurrenz zu Polyethylenterephthalat (PET) tritt. Weitere Beispiele für Kunststoffanwendungen in denen FDCA als Terephthalsäuresubsitut eingesetzt werden kann sind Polybutylenterephthalat (PBT) oder Polybutylenadipat-terephthalat (PBAT). Grundsätzlich kann FDCA in allen Polymeranwendungen verwendet werden, in denen Disäuren als Monomer zum Einsatz kommen.

Durch formale Oxidation der Aldehyd- als auch der Alkohol-Funktion des 2,5-Hydroxymethylfurfural (HMF)(CAS-Nummer 67-47-0) gelangt man in wässriger Lösung über die Zwischenprodukte HFCA (Hydroxymethyl-2-furancarboxylsäure)(CAS-Nummer 6388-41-6) und FFCA (5-Formyl-2-furancarboxylsäure)(CAS-Nummer 13529-17-4) zur 2,5-Furandicarbonsäure (FDCA) (CAS-Nummer 3238-40-2).

Im Stand der Technik sind sowohl homogen als auch heterogen katalytische Oxidationsverfahren für die FDCA-Herstellung aus HMF bekannt. So sind auch Verfahren bekannt, welche HMF zu FDCA mithilfe von Edelmetallkatalysatoren in einem einstufigen Verfahren bei basischen pH-Werten oxidieren. Vor dem Hintergrund, dass die vollständige Oxidation der zweiten Aldehyd-Gruppe von 5-Formyl-2-furancarboxylsäure (FFCA), einem Zwischenprodukt bei der Oxidation von HMF zu FDCA, hohe pH-Werte erfordert, ist es nachteilig, dass HMF aufgrund seiner Reaktivität empfindlich gegen hohe pH-Werte ist. Dementsprechend werden in Verfahren des Standes der Technik häufig pH-Werte eingestellt, welche einen Kompromiss zwischen angestrebtem möglichst hohem Umsatz zu FDCA und zu vermeidender pH-Instabilität des HMF darstellen, dadurch aber auch für beide Bedürfnisse nicht optimal ausgestaltet sind. Darüber hinaus setzen gängige Verfahren zur Oxidation von HMF zu FDCA in der Regel lange Reaktionsdauern voraus, welche wirtschaftlich nachteilig sind. Diese Verfahren werden in der Regel einstufig ausgeführt, das heißt, die für die Umsetzung von HMF erforderliche Einstellung und Aufrechterhaltung der gewünschten pH-Werte unter Sauerstoffzufuhr erfolgt so, dass in einem Verfahrensschritt ohne gezielte Änderung der eingestellten Verfahrensparameter, insbesondere des pH-Werts, FDCA gebildet wird.

KR 101 936 966 B1, US 9,199,957 B2 und JP 2009 029751 A offenbaren Verfahren zur Oxidation von HMF zu FDCA.

Die US 2012/0271060 offenbart ein einstufiges Verfahren zur Herstellung von FDCA aus HMF, wobei bei einer Temperatur von mehr als 140 °C eine HMF-Oxidation bewirkt wird. Ein derartiges Verfahren ist aufgrund der eingesetzten Temperaturen weder wirtschaftlich noch schonend. Die EP 2 601 182 A1 offenbart ein einstufiges Verfahren zur Herstellung von FDCA aus HMF, bei welchem schwache Laugen eingesetzt werden.

Der vorliegenden Erfindung liegt das technische Problem zugrunde, die Nachteile aus dem Stand der Technik zu überwinden und demgemäß insbesondere Verfahren bereitzustellen, welche eine wirtschaftliche Oxidation von HMF zu FDCA ermöglichen, insbesondere bei einer kurzen Reaktionsdauer, erhöhten FDCA-Ausbeuten und/oder besseren Kohlenstoffbilanzen.

Die vorliegende Erfindung löst das ihr zugrundeliegende technische Problem durch die Bereitstellung der Lehren der unabhängigen und abhängigen Ansprüche und der Beschreibung.

Die vorliegende Erfindung stellt insbesondere ein Verfahren zur Herstellung von 2,5-Furandicarboxylsäure (FDCA) aus Hydroxymethylfurfural (HMF) durch, insbesondere 2-stufige, Oxidation des HMF bereit, umfassend die folgenden Verfahrensschritte:
a) Bereitstellen einer wässrigen Lösung enthaltend HMF mit einem pH-Wert in einem Bereich von 7,0 bis 10,0, mindestens einer Lauge und mindestens eines Edelmetallkatalysators,
b) Umsetzen der wässrigen Lösung enthaltend HMF in Anwesenheit mindestens eines gemäß Verfahrensschritt a) bereitgestellten Edelmetallkatalysators unter oxidativen Bedingungen und unter Aufrechterhalten eines konstanten pH-Wertes, der in einem Bereich von 7,0 bis 10,0 liegt, durch Zugabe von 0,7 bis 1,3 Stoffmengenäquivalenten (eq) (bezogen auf Stoffmenge des HMF in der eingesetzten wässrigen Lösung) der mindestens einen Lauge zum Erhalt einer Zwischenproduktlösung enthaltend Hydroxymethyl-2-furancarboxylsäure (HFCA) und/oder 5-Formyl-2-furancarboxylsäure (FFCA) und
c) Umsetzen der Zwischenproduktlösung in einem pH-Wert-Bereich von 10,5 bis 14,0 in Anwesenheit mindestens eines gemäß Verfahrensschritt a) bereitgestellten Edelmetallkatalysators unter oxidativen Bedingungen zum Erhalt einer Lösung enthaltend 2,5-Furandicarboxylsäure (FDCA).

In bevorzugter Ausführungsform betrifft die vorliegende Erfindung ein vorgenanntes Verfahren, wobei Verfahrensschritt c) unter Einstellen und Aufrechterhalten eines konstanten pH-Wertes, insbesondere eines einzigen konstanten pH-Wertes, der in dem Bereich von 10,5 bis 14,0 liegt, durch Zugabe der mindestens einen Lauge durchgeführt wird.

Die vorliegende Erfindung stellt insbesondere ein Verfahren zur Herstellung von 2,5-Furandicarboxylsäure (FDCA) aus Hydroxymethylfurfural (HMF) durch, insbesondere 2-stufige, Oxidation des HMF bereit, umfassend die folgenden Verfahrensschritte:
a) Bereitstellen einer wässrigen Lösung enthaltend HMF mit einem pH-Wert in einem Bereich von 7,0 bis 10,0, mindestens einer Lauge und mindestens eines Edelmetallkatalysators,
b) Umsetzen der wässrigen Lösung enthaltend HMF in Anwesenheit mindestens eines gemäß Verfahrensschritt a) bereitgestellten Edelmetallkatalysators unter oxidativen Bedingungen und unter Aufrechterhalten eines konstanten pH-Wertes, der in einem Bereich von 7,0 bis 10,0 liegt, durch Zugabe von 0,7 bis 1,3 Stoffmengenäquivalenten (eq) (bezogen auf Stoffmenge des HMF in der eingesetzten wässrigen Lösung) der mindestens einen Lauge zum Erhalt einer Zwischenproduktlösung enthaltend Hydroxymethyl-2-furancarboxylsäure (HFCA) und/oder 5-Formyl-2-furancarboxylsäure (FFCA) und
c) Umsetzen der Zwischenproduktlösung in Anwesenheit mindestens eines gemäß Verfahrensschritt a) bereitgestellten Edelmetallkatalysators unter oxidativen Bedingungen und unter Einstellen und Aufrechterhalten eines konstanten pH-Wertes, der in einem Bereich von 10,5 bis 14,0 liegt, durch Zugabe der mindestens einen Lauge zum Erhalt einer Lösung enthaltend 2,5-Furandicarboxylsäure (FDCA).

Demgemäß wird ein Verfahren bereitgestellt, bei dem aus HMF FDCA durch ein, bevorzugt zweistufiges, Verfahren umfassend die Verfahrensschritte (im Folgenden auch kurz "Schritte" genannt) a), b) und c) hergestellt wird. Hierzu wird in einem Verfahrensschritt a) in einer wässrigen Lösung mit einem pH-Wert in einem Bereich von 7,0 bis 10,0 das Ausgangsprodukt HMF, mindestens eine Lauge sowie mindestens ein Edelmetallkatalysator bereitgestellt.

In Verfahrensschritt b), das heißt in einer ersten Stufe, wird anschließend in Anwesenheit des Edelmetallkatalysators HMF, enthalten in der wässrigen Lösung, unter oxidativen Bedingungen unter Aufrechterhalten des in Verfahrensschritt a) bereitgestellten pH-Wertes, also bei einem konstanten pH-Wert im Bereich von 7,0 bis 10,0, durch Zugabe von 0,7 bis 1,3 Stoffmengenäquivalenten (eq) der mindestens einen Lauge umgesetzt, insbesondere oxidiert, zu Hydroxymethyl-2-Furancarboxylsäure (HFCA) und/oder 5-Formyl-2-furancarboxylsäure (FFCA), wobei die Stoffmengenäquivalente bezogen sind auf die Stoffmenge des HMFs in der wässrigen Lösung. Erhalten wird eine Zwischenproduktlösung enthaltend Hydroxymethyl-2-Furancarboxylsäure (HFCA) und/oder 5-Formyl-2-furancarboxylsäure (FFCA) mit einem pH-Wert im Bereich von 7,0 bis 10,0.

Über bevorzugt den gesamten zeitlichen Verlauf des Verfahrensschrittes b) wird die erfindungsgemäße Menge an Stoffmengenäquivalenten der mindestens einen Lauge zur wässrigen Lösung kontinuierlich hinzugefügt, die zum Konstanthalten des pH-Werts notwendig ist.

Anschließend, das heißt nachdem die Zwischenproduktlösung enthaltend HFCA und/oder FFCA erhalten wurde, wobei bevorzugt sämtliches HMF umgesetzt wurde, wird in einem Verfahrensschritt c), das heißt in einer zweiten Stufe, die Zwischenproduktlösung in Anwesenheit des mindestens einen Edelmetallkatalysators unter oxidativen Bedingungen und unter Einstellen und Aufrechterhalten eines pH-Wertes, welcher erfindungsgemäß oberhalb des im vorherigen Verfahrensschritt b) eingesetzten pH-Wertes in einem Bereich von 10,5 bis 14,0 liegt, durch Zugabe weiterer Mengen der mindestens einen Lauge weiter umgesetzt. In diesem Verfahrensschritt c) wird also der pH-Wert der Zwischenproduktlösung aus Verfahrensschritt b) auf einen pH-Wert im Bereich von 10,5 bis 14,0 angehoben und anschließend unter Zugabe weiterer mindestens einen Lauge konstant gehalten, wobei dabei HFCA und/oder FFCA, insbesondere beide, zu FDCA umgesetzt, insbesondere oxidiert, werden. In Verfahrensschritt c) wird HFCA und/oder FFCA zu FDCA umgesetzt und somit in diesem Verfahrensschritt eine Lösung enthaltend FDCA erhalten.

In Verfahrensschritt c) kann bevorzugt die Zugabe der mindestens einen Lauge in Teilen erfolgen, das heißt, dass zunächst am Anfang von Verfahrensschritt c) ein Teil der mindestens einen Lauge zum Anheben des pH-Wertes und anschließend wird ein weiterer Teil kontinuierlich zum Aufrechterhalten eines konstanten pH-Werts über den gesamten zeitlichen Verlauf des Verfahrensschrittes c) zur wässrigen Zwischenproduktlösung hinzugefügt.

In einer bevorzugten Ausführungsform weist das erfindungsgemäße Verfahren einen zusätzlichen Verfahrensschritt d) nach Verfahrensschritt c) auf, in welchem der mindestens eine Edelmetallkatalysator aus der Lösung enthaltend FDCA abgetrennt wird, insbesondere mittels Filtration.

In einer besonders bevorzugten Ausführungsform betrifft die vorliegende Erfindung ein erfindungsgemäßes Verfahren, wobei in einem Verfahrensschritt a0) vor Verfahrensschritt a) der pH-Wert einer wässrigen Lösung, enthaltend HMF, mit einem pH-Wert von 3,0 bis 6,0 auf einen pH-Wert in einem Bereich von 7,0 bis 10,0 erhöht wird, insbesondere unter nicht oxidativer Bedingung, insbesondere ohne Zufuhr von Luft oder Sauerstoff.

In einer besonders bevorzugten Ausführungsform der vorliegenden Erfindung kann in Verfahrensschritt a0) das Erhöhen des pH-Wertes durch Zugabe mindestens einer Lauge erfolgen.

In einer bevorzugten Ausführungsform wird in Verfahrensschritt a) eine Lauge bereitgestellt und diese in beiden Verfahrensschritten b) und c) verwendet, insbesondere in bevorzugter Ausführungsform auch in Verfahrensschritt a0).

In einer bevorzugten Ausführungsform wird in Verfahrensschritt a) eine starke Lauge bereitgestellt und diese in beiden Verfahrensschritten b) und c) verwendet, insbesondere in bevorzugter Ausführungsform auch in Verfahrensschritt a0).

In einer besonders bevorzugten Ausführungsform ist die mindestens eine Lauge NaOH oder KOH.

In einer bevorzugten Ausführungsform werden in Verfahrensschritt a) mindestens zwei unterschiedliche Laugen bereitgestellt, wobei diese Laugen bevorzugt jeweils starke Laugen sind.

In einer bevorzugten Ausführungsform werden in Verfahrensschritt a) mindestens zwei unterschiedliche Laugen bereitgestellt, wobei diese Laugen bevorzugt jeweils eine starke und eine schwache Lauge sind.

In einer bevorzugten Ausführungsform werden in Verfahrensschritt a) mindestens zwei unterschiedliche Laugen bereitgestellt, wobei die erste Lauge in Verfahrensschritt b) und die zweite Lauge in Verfahrensschritt c) verwendet wird.

In einer bevorzugten Ausführungsform werden in Verfahrensschritt a) mindestens zwei unterschiedliche Laugen bereitgestellt, wobei in Verfahrensschritt b) eine erste, schwache Lauge und in Verfahrensschritt c) eine zweite, starke Lauge verwendet werden.

In einer besonders bevorzugten Ausführungsform ist die erste, schwache Lauge, insbesondere verwendet in Verfahrensschritt b) ausgewählt aus der Gruppe bestehend aus Ammoniak, Carbonaten, Hydrogencarbonaten, Formiaten und Acetaten.

In einer besonders bevorzugten Ausführungsform ist die zweite, starke Lauge, insbesondere verwendet in Verfahrensschritt c), NaOH oder KOH.

In einer bevorzugten Ausführungsform weist der in Verfahrensschritt a) bereitgestellte mindestens eine Edelmetallkatalysator mindestens ein Edelmetall auf, insbesondere ausgewählt aus der Gruppe bestehend aus Kupfer, Ruthenium, Kobalt, Iridium, Rhodium, Platin, Palladium, Gold und Silber.

In einer bevorzugten Ausführungsform weist der in Verfahrensschritt a) bereitgestellte mindestens eine Edelmetallkatalysator mindestens ein Edelmetall auf, insbesondere ausgewählt aus der Gruppe bestehend aus Ruthenium, Kobalt, Iridium, Rhodium, Platin, Palladium, Gold und Silber.

In einer besonders bevorzugten Ausführungsform weist der Edelmetallkatalysator mindestens ein Edelmetall, insbesondere ausgewählt aus der Gruppe bestehend aus Kupfer, Ruthenium, Kobalt, Iridium, Rhodium, Platin, Palladium, Gold und Silber, sowie mindestens ein Dotiermittel, insbesondere Blei, Zinn, Thallium, Tellur, Kobalt oder Bismut, auf, insbesondere Bismut.

In einer besonders bevorzugten Ausführungsform weist der Edelmetallkatalysator mindestens ein Edelmetall, insbesondere ausgewählt aus der Gruppe bestehend aus Ruthenium, Kobalt, Iridium, Rhodium, Platin, Palladium, Gold und Silber, sowie mindestens ein Dotiermittel, insbesondere Blei, Zinn, Thallium, Tellur, Kobalt oder Bismut, auf, insbesondere Bismut.

In einer besonders bevorzugten Ausführungsform ist der Edelmetallkatalysator ein Pt-Bi-Edelmetallkatalysator.

In einer besonders bevorzugten Ausführungsform ist der in Verfahrensschritt b) eingesetzte Edelmetallkatalysator ein Gold-Katalysator und der in Verfahrensschritt c) eingesetzte Edelmetallkatalysator ein Pt-Bi-Edelmetallkatalysator, beide Katalysatoren sind vorzugsweise geträgert.

In einer besonders bevorzugten Ausführungsform enthält der Edelmetallkatalysator kein Kupfer.

In einer bevorzugten Ausführungsform weist der in Verfahrensschritt a) bereitgestellte Edelmetallkatalysator mindestens einen Träger auf, insbesondere ausgewählt aus der Gruppe bestehend aus Metalloxiden, insbesondere Magnesiumoxid, Ceroxid, Zirkoniumoxid, Hydroxyapatit, Titandioxid, Hydrotalcit (HAT), Al₂O₃ und Kohlenstoff (C).

In einer besonders bevorzugten Ausführungsform beträgt die eingesetzte Menge an Edelmetallkatalysator, die in Verfahrensschritt a) bereitgestellt wird, 0,1 bis 25, insbesondere 1 bis 25, insbesondere 2 bis 20, insbesondere 5 bis 15, insbesondere 7 bis 14, insbesondere 5 g/l (bezogen auf das Volumen der wässrigen Lösung enthaltend HMF).

In einer besonders bevorzugten Ausführungsform beträgt die eingesetzte Menge an HMF, die in Verfahrensschritt a) bereitgestellt wird, 0,1 bis 2,5, insbesondere 0,2 bis 2,0, insbesondere 0,3 bis 1,5, insbesondere 0,4 bis 0,8, insbesondere 0,4 mol/l (bezogen auf das Volumen der wässrigen Lösung enthaltend HMF).

In einer bevorzugten Ausführungsform werden in Verfahrensschritt b) 0,8 bis 1,2, insbesondere 0,9 bis 1,1 Stoffmengenäquivalente (eq) (bezogen auf Stoffmenge des HMF in der eingesetzten wässrigen Lösung) der mindestens einen Lauge zugegeben.

In einer bevorzugten Ausführungsform werden in Verfahrensschritt c) 0,7 bis 1,3, insbesondere 0,8 bis 1,2, insbesondere 0,9 bis 1,1 Stoffmengenäquivalente (eq) (bezogen auf Stoffmenge des HMF in der eingesetzten wässrigen Lösung) der mindestens einen Lauge zugegeben.

In einer besonders bevorzugten Ausführungsform der vorliegenden Erfindung wird in Verfahrensschritt c) der gleiche mindestens eine Edelmetallkatalysator wie im vorherigen Verfahrensschritt b) verwendet, insbesondere wird in Verfahrensschritt c) der in Verfahrensschritt b) verwendete mindestens eine Edelmetallkatalysator verwendet.

In einer besonders bevorzugten Ausführungsform der vorliegenden Erfindung wird der in Verfahrensschritt b) eingesetzte mindestens eine Edelmetallkatalysator nicht von der Zwischenproduktlösung enthaltend Hydroxymethyl-2-furancarboxylsäure (HFCA) und/oder 5-Formyl-2-furancarboxylsäure (FFCA) abgetrennt und so auch in Verfahrensschritt c) eingesetzt.

In einer besonders bevorzugten Ausführungsform der vorliegenden Erfindung wird in Verfahrensschritt c) ein anderer Edelmetallkatalysator als im vorherigen Verfahrensschritt b) verwendet.

In einer bevorzugten Ausführungsform werden in Verfahrensschritt a) zwei Edelmetallkatalysatoren bereitgestellt, wobei in Verfahrensschritt b) ein erster Edelmetallkatalysator und in Verfahrensschritt c) ein zweiter Edelmetallkatalysator verwendet werden, wobei der erste Edelmetallkatalysator ungleich dem zweiten Edelmetallkatalysator ist.

In einer bevorzugten Ausführungsform werden in Verfahrensschritt a) zwei Edelmetallkatalysatoren bereitgestellt, wobei in Verfahrensschritt b) ein erster Edelmetallkatalysator und in Verfahrensschritt c) ein zweiter Edelmetallkatalysator verwendet werden, wobei der erste Edelmetallkatalysator ein anderes Edelmetall als der zweite Edelmetallkatalysator aufweist.

In einer bevorzugten Ausführungsform werden in Verfahrensschritt a) zwei Edelmetallkatalysatoren oder mehr Edelmetallkatalysatoren bereitgestellt, wobei in Verfahrensschritt b) ein erster Edelmetallkatalysator und in Verfahrensschritt c) ein anderer Edelmetallkatalysator verwendet werden, wobei der erste Edelmetallkatalysator ungleich dem anderen Edelmetallkatalysator ist.

In einer bevorzugten Ausführungsform werden in Verfahrensschritt a) zwei oder mehr Edelmetallkatalysatoren bereitgestellt, wobei in Verfahrensschritt b) ein erster Edelmetallkatalysator und in Verfahrensschritt c) ein anderer Edelmetallkatalysator verwendet werden, wobei der erste Edelmetallkatalysator ein anderes Edelmetall als der andere Edelmetallkatalysator aufweist.

In einer besonders bevorzugten Ausführungsform sind die oxidativen Bedingungen, insbesondere während der Verfahrensschritte b) und/oder c), Reaktionsbedingungen, die zu einer Zufuhr von Sauerstoff oder Luft in die wässrige Lösung führen, insbesondere durch eine Luft- oder Sauerstoffbegasungsvorrichtung, insbesondere einen Begasungsrührer oder ein Gebläse oder/und einen Strömungskanal, insbesondere unter gleichzeitiger mechanischer Agitation der Lösung, zum Beispiel durch ein Rührwerk oder eine Verwirbelungseinrichtung.

In einer besonders bevorzugten Ausführungsform sind die oxidativen Bedingungen, insbesondere während der Verfahrensschritte b) und/oder c), insbesondere b) und c), die Zufuhr von Sauerstoff in die Lösung durch eine Luft- oder Sauerstoffbegasungsvorrichtung, insbesondere durch eine Luft- oder Sauerstoffbegasungsvorrichtung mit einer Flussrate von 10 bis 5000, insbesondere 100 bis 5000, insbesondere 400 bis 2000, insbesondere 600 bis 1500, insbesondere 1000 ml/min pro Liter Reaktionsvolumen.

In einer besonders bevorzugten Ausführungsform sind die oxidativen Bedingungen, insbesondere während der Verfahrensschritte b) und/oder c), insbesondere b) und c), die Zufuhr von Luft in die Lösung durch eine Luft- oder Sauerstoffbegasungsvorrichtung, insbesondere durch eine Luft- oder Sauerstoffbegasungsvorrichtung mit einer Flussrate von 50 bis 25000, insbesondere 500 bis 25000, insbesondere 2000 bis 10000, insbesondere 3000 bis 7500, insbesondere 5000 ml/min pro Liter Reaktionsvolumen.

In einer besonders bevorzugten Ausführungsform sind die oxidativen Bedingungen, insbesondere während der Verfahrensschritte b) und/oder c), insbesondere b) und c), die Zufuhr von Sauerstoff in die Lösung durch eine Luft- oder Sauerstoffbegasungsvorrichtung, insbesondere mit einer Flussrate von 10 bis 5000, insbesondere 100 bis 5000, insbesondere 400 bis 2000, insbesondere 600 bis 1500, insbesondere 1000 ml/min pro Liter Reaktionsvolumen, und eine mechanische Agitation der Lösung, zum Beispiel durch ein Rührwerk oder eine Verwirbelungseinrichtung.

In einer besonders bevorzugten Ausführungsform sind die oxidativen Bedingungen, insbesondere während der Verfahrensschritte b) und/oder c), insbesondere b) und c), die Zufuhr von Luft in die Lösung durch eine Luft- oder Sauerstoffbegasungsvorrichtung, insbesondere mit einer Flussrate von 50 bis 25000, insbesondere 500 bis 25000, insbesondere 2000 bis 10000, insbesondere 3000 bis 7500, insbesondere 5000 ml/min Luft pro Liter Reaktionsvolumen, und eine mechanische Agitation der Lösung, zum Beispiel durch ein Rührwerk oder eine Verwirbelungseinrichtung.

In einer besonders bevorzugten Ausführungsform werden die oxidativen Bedingungen in den Verfahrensschritte b), c) oder b) und c) mit Hilfe eines Begasungsrührers bereitgestellt.

Ein erfindungsgemäß bevorzugt in den Schritten b) und c) verwendeter Begasungsrührer stellt sowohl die Funktionen einer Luft- oder Sauerstoffbegasungsvorrichtung als auch eines Rührwerks bereit.

In einer bevorzugten Ausführungsform werden die Verfahrensschritte b) und/oder c) unter mechanischer Agitation, insbesondere unter Rühren oder Verwirbeln, durchgeführt, insbesondere mittels eines Begasungsrührers.

In einer bevorzugten Ausführungsform wird die wässrige Lösung in Verfahrensschritt b) mittels des Rührwerks gerührt, insbesondere mit einer Geschwindigkeit von 50 bis 5000, insbesondere 200 bis 1000, insbesondere 300 bis 900, insbesondere 400 bis 500 U/min (Umdrehung der Rührwelle pro Minute, auch als upm bezeichnet).

In einer bevorzugten Ausführungsform wird die wässrige Lösung in Verfahrensschritt c) mittels des Rührwerks gerührt, insbesondere mit einer Geschwindigkeit von 50 bis 5000, insbesondere 200 bis 1000, insbesondere 300 bis 900, insbesondere 400 bis 500 U/min (Umdrehung der Rührwelle pro Minute).

In einer bevorzugten Ausführungsform wird die wässrige Lösung in Verfahrensschritten b) und c) mittels des Rührwerks gerührt, insbesondere mit einer Geschwindigkeit von 50 bis 5000, insbesondere 200 bis 1000, insbesondere 300 bis 900, insbesondere 400 bis 500 U/min (Umdrehung der Rührwelle pro Minute).

In einer besonders bevorzugten Ausführungsform werden die Verfahrensschritte a0), a), b) und c), insbesondere a), b) und c), insbesondere b) und c) bei einem Druck von Normaldruck bis 20 bar, insbesondere von Normaldruck bis 10 bar, insbesondere von Normaldruck bis 5 bar durchgeführt.

In einer besonders bevorzugten Ausführungsform werden die Verfahrensschritte a0), a), b) und c), insbesondere a), b) und c), insbesondere b) und c) bei Normaldruck durchgeführt.

In einer bevorzugten Ausführungsform werden Verfahrensschritte b) oder c) oder beide Verfahrensschritte bei einer Temperatur von 40 bis 100 °C durchgeführt, insbesondere bei einer Temperatur von 50 bis 90 °C, insbesondere bei einer Temperatur von 55 bis 80 °C, insbesondere von 55 bis 65 °C, insbesondere bei 60 °C.

In einer bevorzugten Ausführungsform weist das erfindungsgemäße Verfahren nach Verfahrensschritt c) oder d) einen zusätzlichen Verfahrensschritt f) auf, in welchem das in Verfahrensschritt c) bevorzugt als Anion, also nicht protoniertes FDCA, erhaltene FDCA protoniert wird, insbesondere, indem die Lösung enthaltend FDCA auf einen pH-Wert von 1,0 bis 6,5 eingestellt und FDCA ausgefällt wird.

In einer bevorzugten Ausführungsform weist das erfindungsgemäße Verfahren nach Verfahrensschritt c) oder d) einen zusätzlichen Verfahrensschritt f) auf, in welchem die Lösung enthaltend FDCA auf einen pH-Wert von 1,0 bis 6,5 eingestellt, FDCA ausgefällt und protoniertes FDCA erhalten wird.

In einer bevorzugten Ausführungsform weist das erfindungsgemäße Verfahren einen zusätzlichen Verfahrensschritt e) nach Verfahrensschritt c) oder d) und vorzugsweise vor Verfahrensschritt f), insbesondere nach Verfahrensschritt d) und vor Verfahrensschritt f), auf, in welchem die Lösung enthaltend FDCA aufgereinigt wird.

In einer bevorzugten Ausführungsform weist das erfindungsgemäße Verfahren einen zusätzlichen Verfahrensschritt g) nach Verfahrensschritt c) oder d) oder f), insbesondere nach d) oder f), auf, in welchem protoniertes (nach Verfahrensschritt f)) oder nicht-protoniertes FDCA (nach Verfahrensschritt c) oder d)) aufgereinigt wird, bevorzugt mittels Waschung, Umkristallisation, Schmelzen, Adsorption oder einer Kombination davon.

In einer bevorzugten Ausführungsform weist das erfindungsgemäße Verfahren einen zusätzlichen Verfahrensschritt g) nach Verfahrensschritt f), auf, in welchem protoniertes oder nicht-protoniertes FDCA aufgereinigt wird, bevorzugt mittels Waschung, Umkristallisation, Schmelzen, Adsorption oder einer Kombination davon.

In einer bevorzugten Ausführungsform wird gemäß Verfahrensschritt g) isoliertes FDCA erhalten.

**In** einer bevorzugten Ausführungsform weist das erfindungsgemäße Verfahren einen zusätzlichen Verfahrensschritt h) nach Verfahrensschritt c), d), e), f) oder g) auf, in welchem protoniertes oder nicht-protoniertes FDCA in einen Methyl-, Ethyl- oder Dimethylester überführt wird.

Die vorliegende Erfindung betrifft auch Verfahren zur Herstellung von Polyestern, Polyamiden oder Polyurethanen, insbesondere Polyethylenfuranoat (PEF), wobei dieses Verfahren ein erfindungsgemäßes Verfahren zur Herstellung von FDCA und anschließend ein Polymerisieren des aus Verfahrensschritt c), d), e), f) oder g) erhaltenen, vorzugsweise nach Verfahrensschritt d), e), f) oder g) erhaltenen FDCA umfasst.

In einer bevorzugten Ausführungsform umfasst das Verfahren zur Herstellung von Polyestern, Polyamiden oder Polyurethanen eine Aufreinigung des aus Verfahrensschritt c), d) oder f), vorzugsweise d) oder f), erhaltenen FDCA vor dem Polymerisieren, insbesondere gemäß Verfahrensschritt g).

In einer besonders bevorzugten Ausführungsform besteht das erfindungsgemäße Verfahren aus den Verfahrensschritten a), b), und c), insbesondere werden zwischen diesen Verfahrensschritten keine weiteren Verfahrensschritte durchgeführt.

In einer bevorzugten Ausführungsform werden zwischen den Verfahrensschritten b) und c) keine weiteren Verfahrensschritte durchgeführt.

In einer bevorzugten Ausführungsform wird zwischen den Verfahrensschritten b) und c) kein Aufreinigungsschritt durchgeführt.

In einer bevorzugten Ausführungsform wird die in Verfahrensschritt b) erhaltene Zwischenproduktlösung enthaltend Hydroxymethyl-2-furancarboxylsäure (HFCA) und/oder 5-Formyl-2-furancarboxylsäure (FFCA) ohne weitere Verfahrensschritte, insbesondere Aufreinigungsschritte und/oder Abtrennungsschritte, insbesondere Filtrationsschritte, in Verfahrensschritt c) umgesetzt.

In einer bevorzugten Ausführungsform weist das Verfahren nach dem Verfahrensschritt b) und vor dem Verfahrensschritt c) einen Verfahrensschritt b1) auf, in welchem der mindestens eine Edelmetallkatalysator aus der Zwischenproduktlösung enthaltend Hydroxymethyl-2-furancarboxylsäure (HFCA) und/oder 5-Formyl-2-furancarboxylsäure (FFCA) abgetrennt wird, insbesondere mittels Filtration.

In einer besonders bevorzugten Ausführungsform weist das Verfahren nach dem Verfahrensschritt b1) und vor dem Verfahrensschritt c) einen Verfahrensschritt b2) auf, in welchem der mindestens eine Edelmetallkatalysator, insbesondere der mindestens eine Edelmetallkatalysator ungleich dem in Verfahrensschritt b1) abgetrennten mindestens einen Edelmetallkatalysator, zu der Zwischenproduktlösung enthaltend Hydroxymethyl-2-furancarboxylsäure (HFCA) und/oder 5-Formyl-2-furancarboxylsäure (FFCA) zugegeben wird.

Im Zusammenhang mit der vorliegenden Erfindung wird unter einer "Abtrennung" beziehungsweise einem "Abtrennungsschritt" ein Abtrennen mindestens eines Katalysators aus einer Lösung, insbesondere Zwischenproduktlösung enthaltend Hydroxymethyl-2-furancarboxylsäure (HFCA) und/oder 5-Formyl-2-furancarboxylsäure (FFCA) oder Lösung enthaltend 2,5-Furandicarboxylsäure (FDCA), verstanden, wobei die Zusammensetzung der Lösung, insbesondere Zwischenproduktlösung enthaltend Hydroxymethyl-2-furancarboxylsäure (HFCA) und/oder 5-Formyl-2-furancarboxylsäure (FFCA) oder Lösung enthaltend 2,5-Furandicarboxylsäure (FDCA), unverändert bleibt. Bevorzugt liegen nach der Abtrennung beziehungsweise nach dem Abtrennungsschritt die in der Lösung, insbesondere Zwischenproduktlösung enthaltend Hydroxymethyl-2-furancarboxylsäure (HFCA) und/oder 5-Formyl-2-furancarboxylsäure (FFCA) oder Lösung enthaltend 2,5-Furandicarboxylsäure (FDCA), enthaltenen Stoffe, insbesondere Edukte, Produkte und Nebenprodukte, im gleichen Verhältnis, insbesondere Volumen- und/oder Stoffmengenverhältnis, zueinander vor, wie vor der Abtrennung beziehungsweise vor dem Abtrennungsschritt.

Im Zusammenhang mit der vorliegenden Erfindung ist eine "Abtrennung" beziehungsweise ein "Abtrennungsschritt" mindestens eines Katalysators aus einer Lösung, insbesondere Zwischenproduktlösung enthaltend Hydroxymethyl-2-furancarboxylsäure (HFCA) und/oder 5-Formyl-2-furancarboxylsäure (FFCA) oder Lösung enthaltend 2,5-Furandicarboxylsäure (FDCA), keine Aufreinigung bzw. kein Aufreinigungsschritt eines in einer Lösung, insbesondere Zwischenproduktlösung enthaltend Hydroxymethyl-2-furancarboxylsäure (HFCA) und/oder 5-Formyl-2-furancarboxylsäure (FFCA) oder Lösung enthaltend 2,5-Furandicarboxylsäure (FDCA) enthaltenen Stoffes, insbesondere Zwischenprodukts oder Produkts, insbesondere Hydroxymethyl-2-furancarboxylsäure (HFCA) und/oder 5-Formyl-2-furancarboxylsäure (FFCA) oder 2,5-Furandicarboxylsäure (FDCA).

Im Zusammenhang mit der vorliegenden Erfindung wird unter "Aufreinigung" beziehungsweise "Aufreinigungsschritt" ein Entfernen von Edukten und/oder Nebenprodukten aus einem Gemisch enthaltend ein Produkt und die Edukte und/oder Nebenprodukte verstanden. Bevorzugt wird unter Aufreinigung beziehungsweise Aufreinigungsschritt ein Entfernen von Edukten, insbesondere HMF, Zwischenprodukten, insbesondere Hydroxymethyl-2-furancarboxylsäure (HFCA) und/oder 5-Formyl-2-furancarboxylsäure (FFCA), und/oder Nebenprodukten von 2,5-Furandicarboxylsäure (FDCA) verstanden. Bevorzugt ist die Aufreinigung oder der Aufreinigungsschritt eine Waschung, eine Umkristallisation, ein Schmelzen, eine Adsorption, eine Extraktion, eine Destillation oder eine Kombination davon.

In besonders bevorzugter Ausführungsform der vorliegenden Erfindung umfasst das erfindungsgemäße Verfahren die Verfahrensschritte a), b), und c), wobei zwischen den Verfahrensschritten a), b) und c) keine weiteren Verfahrensschritte durchgeführt werden, optional allerdings vor Durchführung von Verfahrensschritt a) und/oder nach Durchführung von Verfahrensschritt c) weitere Verfahrensschritte durchgeführt werden, insbesondere Verfahrensschritt a0) vor Verfahrensschritt a) und wenigstens einer der Verfahrensschritte d) bis g) nach Verfahrensschritt c).

Erfindungsgemäß umfasst das vorliegende Verfahren die Verfahrensschritte a) bis c), bevorzugt a0) bis c), insbesondere a) bis d), bevorzugt a0) bis d), bevorzugt a) bis e), bevorzugt a0) bis e), insbesondere a) bis f), bevorzugt a0) bis f), bevorzugt a) bis g), bevorzugt a0) bis g). Erfindungsgemäß besonders bevorzugt umfasst das vorliegende Verfahren die Verfahrensschritte a0), a), b), c), d), e) und f). Es kann jedoch auch vorgesehen sein, dass das vorliegende Verfahren die Schritte a), b), c), d), e) und f) umfasst. Erfindungsgemäß besonders bevorzugt umfasst das vorliegende Verfahren die Verfahrensschritte a0), a), b), c), d), e), f) und g). Erfindungsgemäß besonders bevorzugt umfasst das vorliegende Verfahren die Verfahrensschritte a), b), c), d), e), f) und g).

In besonders bevorzugter Ausführungsform besteht das vorliegende Verfahren aus den Verfahrensschritten a) bis c), bevorzugt a) bis d), bevorzugt a) bis e), bevorzugt a) bis f), bevorzugt a0) bis c), insbesondere a0) bis d), insbesondere a0) bis e), insbesondere a0) bis f), bevorzugt a0) bis g), bevorzugt a) bis g).

In bevorzugter Ausführungsform wird das Verfahren in der Reihenfolge der Verfahrensschritte a), b), c), und sofern optional vorgesehen, d), e) und f) durchgeführt, insbesondere a0), a), b), c) und sofern optional vorgesehen, d), e), f) und g).

Erfindungsgemäß bevorzugt erfolgt in einer Ausführungsform im Verfahren zur Herstellung von FDCA gemäß zumindest der Verfahrensschritte a) bis c) die Umsetzung von HMF zu FDCA, in einem Batch-Verfahren, das heißt unter batchweiser Zuführung von Edukten und Entnahme von Produkten, vorzugsweise in einem batch-Reaktorsystem, insbesondere einem Reaktor.

Erfindungsgemäß erfolgt in einer Ausführungsform im Verfahren zur Herstellung von FDCA gemäß zumindest der Schritte a) bis c) die Umsetzung von HMF zu FDCA in kontinuierlicher Verfahrensweise, das heißt unter konstanter Zuführung von Edukten und Entnahme von Produkten, vorzugsweise in einem kontinuierlichen Reaktorsystem.

Gemäß der vorliegenden Erfindung werden weder HMF noch die Zwischenprodukte und FDCA während des Verfahrens in einem anderen Lösungsmittel als Wasser, insbesondere nicht in einem organischen Lösungsmittel, eingesetzt, umgesetzt oder hergestellt. Das erfindungsgemäße Verfahren ist bevorzugt frei vom Einsatz von organischen Lösungsmitteln.

In bevorzugter Ausführungsform wird Wasser als alleiniges Lösungsmittel im erfindungsgemäßen Verfahren verwendet.

Die vorliegende Erfindung stellt daher insbesondere ein Verfahren zur Herstellung von 2,5-Furandicarboxylsäure (FDCA) aus Hydroxymethylfurfural (HMF) durch, insbesondere 2-stufige, Oxidation des HMF bereit, umfassend die folgenden Verfahrensschritte:
a) Bereitstellen einer wässrigen Lösung enthaltend HMF mit einem pH-Wert in einem Bereich von 7,0 bis 10,0, mindestens einer Lauge und mindestens eines Edelmetallkatalysators,
b) Umsetzen der wässrigen Lösung enthaltend HMF in Anwesenheit des mindestens einen Edelmetallkatalysators unter oxidativen Bedingungen und unter Aufrechterhalten eines konstanten pH-Wertes, der in einem Bereich von 7,0 bis 10,0 liegt, durch Zugabe von 0,7 bis 1,3 Stoffmengenäquivalenten (eq) (bezogen auf Stoffmenge des HMF in der eingesetzten wässrigen Lösung) der mindestens einen Lauge zum Erhalt einer Zwischenproduktlösung enthaltend Hydroxymethyl-2-furancarboxylsäure (HFCA) und/oder 5-Formyl-2-furancarboxylsäure (FFCA) und
c) Umsetzen der Zwischenproduktlösung in Anwesenheit des mindestens einen Edelmetallkatalysators unter oxidativen Bedingungen und unter Einstellen und Aufrechterhalten eines konstanten pH-Wertes, der in einem Bereich von 10,5 bis 14,0 liegt, durch Zugabe der mindestens einen Lauge zum Erhalt einer Lösung enthaltend 2,5-Furandicarboxylsäure (FDCA).

Im Zusammenhang mit der vorliegenden Erfindung wird unter Normaldruck der Gasdruck verstanden, der einem mittleren Luftdruck an der Erdoberfläche von 101 325 Pa = 1,013 25 bar entspricht.

Im Zusammenhang mit der vorliegenden Erfindung wird unter einem "Rührwerk" eine Rührvorrichtung verstanden, also eine Vorrichtung, die ein an mindestens einer Rührwelle angeordnetes Rührelement aufweist und dazu ausgebildet ist, eine Lösung zu rühren.

Im Zusammenhang mit der vorliegenden Erfindung wird unter einer "Verwirbelungseinrichtung" eine Vorrichtung verstanden, die so ausgebildet ist, dass sie in einer Lösung Strömungsbewegungen verursachen kann.

Im Zusammenhang mit der vorliegenden Erfindung wird unter einer "schwachen Lauge" eine Lauge mit einem pKS von 10,4 bis 3,75 verstanden.

Im Zusammenhang mit der vorliegenden Erfindung wird unter einer "starken Lauge" eine Lauge mit einem pKb von < 3 verstanden.

Im Zusammenhang mit der vorliegenden Erfindung wird unter dem Begriff "Stoffmengenäquivalente (eq)" die Stoffmenge in mol verstanden, welche der Stoffmenge entspricht, auf welche Bezug genommen wird. Liegen beispielsweise in einer Lösung mit einem Volumen von 2 L 3 mol von Stoff A (Konzentration A 1,5 mol/L) vor und es sollen 0,5 Stoffmengenäquivalente von Stoff B zugegeben werden, entsprechen diese 0,5 eq also 1,5 mol, die der Lösung hinzuzufügen sind (Konzentration B 0,75 mol/L).

Im Zusammenhang mit der vorliegenden Erfindung werden unter "oxidativen Bedingungen" Reaktionsbedingungen oder Reaktionsumgebungen verstanden, die insbesondere durch die Verfahrensparameter Temperatur, pH, Druck und Sauerstoffanwesenheit so charakterisiert sind, dass eine Oxidation eines Ausgangsstoffes, insbesondere HMF, oder Zwischenproduktes, insbesondere HFCA und/oder FFCA, ermöglicht wird, insbesondere während der Verfahrensschritte b) und/oder c). Im Zusammenhang mit der vorliegenden Erfindung werden unter "oxidativen Bedingungen" insbesondere Reaktionsbedingungen verstanden, die durch eine Zufuhr von Sauerstoff oder Luft in die wässrige Lösung gekennzeichnet sind, zum Beispiel durch eine Luft- oder Sauerstoff-Begasungsvorrichtung, wie einen Begasungsrührer oder ein Gebläse oder/und einen Strömungskanal, insbesondere unter gleichzeitiger mechanischer Agitation der Lösung, zum Beispiel durch ein Rührwerk oder eine Verwirbelungseinrichtung.

Im Zusammenhang mit der vorliegenden Erfindung werden unter "nicht oxidativen Bedingungen" Reaktionsbedingungen oder Reaktionsumgebungen verstanden, die insbesondere durch die Verfahrensparameter Temperatur, pH, Druck und Sauerstoffanwesenheit so charakterisiert sind, dass eine Oxidation eines Ausgangsstoffes, insbesondere HMF oder Zwischenproduktes, insbesondere HFCA und/oder FFCA, nicht ermöglicht wird, insbesondere während der Verfahrensschritte b) und/oder c). Im Zusammenhang mit der vorliegenden Erfindung werden unter "nicht oxidativen Bedingungen" insbesondere Reaktionsbedingungen verstanden, die nicht durch eine Zufuhr von Sauerstoff oder Luft in die wässrige Lösung gekennzeichnet sind.

Im Zusammenhang mit der vorliegenden Erfindung wird unter einer "Zufuhr" von Luft oder Sauerstoff ein durch geeignete Verfahrensmaßnahmen und insbesondere unter Einsatz von Luftoder Sauerstoff-Begasungsvorrichtungen, insbesondere einem Begasungsrührer, gezielt herbeigeführter Zustrom, das heißt lokale Konzentrationserhöhung, von Luft oder Sauerstoff in der wässrigen Lösung verstanden, der über reine Diffusionsprozesse hinausgeht. Diese Zufuhr ist vorzugsweise eine kontinuierliche Zufuhr. Die Zufuhr dient dazu, den in wässriger Lösung vorliegenden Sauerstoff, der durch die Reaktion verbraucht wird, zu kompensieren, so dass es nicht zu einer Verarmung von Sauerstoff in der wässrigen Lösung kommt.

Im Zusammenhang mit der vorliegenden Erfindung wird unter "Luft" ein Gasgemisch, verstanden, welches einen Volumenanteil von Sauerstoff von 20 bis 22, insbesondere ungefähr 21 Vol.-%, insbesondere 21 Vol.-% (bezogen auf das Gesamtvolumen der Luft) aufweist.

Im Zusammenhang mit der vorliegenden Erfindung wird unter einem "2-stufigen" oder "zweistufigen" Verfahren ein Verfahren verstanden, welches gekennzeichnet ist durch einen Anfangszustand, bei dem die mindestens eine gewünschte chemische Reaktion startet und die anfänglichen Reaktionsbedingungen bevorzugt eingestellt werden oder sind, und einen Endzustand, bei dem die gewünschte, insbesondere maximale, Menge an Ausgangsstoff umgesetzt wurde, wobei zwischen Anfangs- und Endzustand mindestens einmal die Reaktionsbedingungen, insbesondere der pH-Wert, gezielt durch einen Operator, menschlich oder künstlich, geändert wurden. Im Vergleich dazu ist ein einstufiges Verfahren dadurch gekennzeichnet, dass zwischen Anfangs- und Endzustand der pH-Wert nicht, insbesondere überhaupt keine Reaktionsbedingungen, durch einen Operator gezielt geändert wurde.

Im Zusammenhang mit der vorliegenden Erfindung wird unter dem Begriff "konstanter" pH-Wert oder "Aufrechterhalten eines konstanten pH-Wertes" verstanden, dass der jeweilig genannte konkrete pH-Wert mit einer maximalen Abweichung von +/- 0,2 pH-Werteinheiten konstant zu halten ist, insbesondere dass der jeweilig genannte konkrete pH-Wert mit einer maximalen Abweichung von +/- 0,1 pH-Werteinheiten konstant zu halten ist, insbesondere exakt den angegebenen pH-Wert aufweist.

Im Zusammenhang mit der vorliegenden Erfindung wird der pH-Wert wie folgt bestimmt:
Zur pH-Wert-Einstellung und -Regelung wird beispielsweise ein Titrator, zum Beispiel Dulcometer PHD der Firma Prominent, verwendet, welcher die pH-Wertmessung und Laugedosierung in sich vereint. Als pH-Elektrode wird beispielsweise eine pH-Elektrode von Mettler Toledo HA 405-DXK-S8/325 oder eine vergleichbare Elektrode eines anderen Herstellers verwendet. Die pH-Elektrode wird vor dem Einsatz mit 2 Pufferlösungen (zum Beispiel WTW technische Puffer) mit pH 7,00 und pH 10,01 bei 25 °C kalibriert. An den Titrator wird ein Pt-100-Element zur Temperaturkompensation angeschlossen, da die Reaktion bei höheren Temperaturen als 25 °C durchgeführt wird.

Im Zusammenhang mit der vorliegenden Erfindung wird unter "protoniertem FDCA" 2,5-Furandicarbonsäure, CAS-Nummer 3238-40-2, verstanden.

Im Zusammenhang mit der vorliegenden Erfindung wird unter "nicht-protoniertem FDCA" ein ein- oder zweiwertiges Anion der 2,5-Furandicarbonsäure verstanden.

Im Zusammenhang mit der vorliegenden Erfindung wird unter "FDCA" 2,5-Furandicarbonsäure , CAS-Nummer 3238-40-2, verstanden, die sofern sie in Lösung vorliegt, je nach pH-Wert der FDCA enthaltenden Lösung in protonierter oder nicht-protonierter Form vorliegen kann.

Im Zusammenhang mit der vorliegenden Erfindung wird unter dem Begriff "mindestens eine" eine Mengenangabe verstanden, die eine Anzahl von 1 oder 2 oder 3 oder 4 oder 5 oder 6 oder 7 oder 8 oder 9 oder 10 und so weiter ausdrückt. In einer besonders bevorzugten Ausführungsform kann die Bezeichnung "mindestens eine" genau die Anzahl 1 darstellen. In einer weiteren bevorzugten Ausführungsform kann die Begrifflichkeit "mindestens eine" auch 2 oder 3 oder 4 oder 5 oder 6 oder 7 bedeuten.

Im Zusammenhang mit der vorliegenden Erfindung wird unter dem Begriff "mindestens einem Edelmetallkatalysator" eine numerisch definierte Anzahl von Edelmetallkatalysatoren verstanden, wobei die Anzahl 1, 2, 3, 4, 5, 6, 7, 8, 9, 10 oder mehr sein kann.

Sofern im Zusammenhang mit der vorliegenden Erfindung quantitative Angaben, insbesondere Prozentangaben, von Komponenten eines Produktes oder einer Zusammensetzung angegeben sind, addieren diese, sofern nicht explizit anders angegeben oder fachmännisch ersichtlich, zusammen mit den anderen explizit angegeben oder fachmännisch ersichtlichen weiteren Komponenten der Zusammensetzung oder des Produktes auf 100 % der Zusammensetzung und/oder des Produktes auf.

Sofern im Zusammenhang mit der vorliegenden Erfindung ein "Vorhandensein", ein "Enthalten", ein "Aufweisen" oder ein "Gehalt" einer Komponente ausdrücklich erwähnt oder impliziert wird bedeutet dies, dass die jeweilige Komponente vorhanden ist, insbesondere in messbarer Menge vorhanden ist.

Die Zahl der angegebenen Nachkommastellen entspricht der Präzision der jeweils angewandten Messmethode.

Sofern im Zusammenhang mit der vorliegenden Erfindung die erste und zweite Nachkommastelle oder die zweite Nachkommastelle nicht angegeben sind/ist, sind/ist diese als 0 zu setzen.

Unter dem Begriff "und/oder" wird in Zusammenhang mit der vorliegenden Erfindung verstanden, dass alle Mitglieder einer Gruppe, welche durch den Begriff "und/oder" verbunden sind, sowohl alternativ zueinander als auch jeweils untereinander kumulativ in einer beliebigen Kombination offenbart sind. Dies bedeutet für den Ausdruck "A, B und/oder C", dass folgender Offenbarungsgehalt darunter zu verstehen ist: a) A oder B oder C oder b) (A und B), oder c) (A und C), oder d) (B und C), oder e) (A und B und C).

Im Zusammenhang mit der vorliegenden Erfindung wird unter den Begriffen "umfassend" und "aufweisend" verstanden, dass zusätzlich zu den von diesen Begriffen explizit erfassten Elementen noch weitere, nicht explizit genannte Elemente hinzutreten können. Im Zusammenhang mit der vorliegenden Erfindung wird unter diesen Begriffen auch verstanden, dass allein die explizit genannten Elemente erfasst werden und keine weiteren Elemente vorliegen. In dieser besonderen Ausführungsform ist die Bedeutung der Begriffe "umfassend" und "aufweisend" gleichbedeutend mit dem Begriff "bestehend aus". Darüber hinaus erfassen die Begriffe "umfassend" und "aufweisend" auch Zusammensetzungen, die neben den explizit genannten Elementen auch weitere nicht genannte Elemente enthalten, die jedoch von funktioneller und qualitativ untergeordneter Natur sind. In dieser Ausführungsform sind die Begriffe "umfassend" und "aufweisend" gleichbedeutend mit dem Begriff "im Wesentlichen bestehend aus". Der Begriff "bestehend aus" bedeutet, dass allein die explizit genannten Elemente vorliegen und die Anwesenheit weiterer Elemente ausgeschlossen ist.

Weitere Ausführungsformen der vorliegenden Erfindung sind die Gegenstände der Unteransprüche und weiteren unabhängigen Ansprüche.

Die Erfindung wird anhand der folgenden Beispiele und der dazugehörigen Figuren näher erläutert.

Die Figuren zeigen:
Figur 1: NaOH-Verbrauchskurven in Abhängigkeit vom in Verfahrensschritt b) eingestellten pH-Wert für die Versuche 5a - f.
Figur 2: C-Bilanz in Abhängigkeit vom in Verfahrensschritt b) eingestellten pH-Wert für die Versuche 5a - f.
Figur 3: NaOH-Verbrauchskurven in Abhängigkeit vom in Verfahrensschritt c) eingestellten pH-Wert für die Versuche 6a- 6d.
Figur 4: Selektivität für Intermediate und HMF sowie C-Bilanz in Abhängigkeit vom in Verfahrensschritt c) eingestellten pH-Wert für die Versuche 6a - 6d.
Figur 5: NaOH-Verbrauchskurve bei erfindungsgemäßer Verfahrensweise unter Einsatz zweier unterschiedlicher Katalysatoren.

### BEISPIELE

### Beispiel 1 - einstufiges Verfahren (Vergleichsverfahren)

Als Katalysator wurde ein Aktivkohle geträgerter Platin/Bismuth-Katalysator (3,5 %Pt/1 % Bi/C (=Norit SX UltraCat)) eingesetzt. Die HMF-Oxidation erfolgte unter folgenden Reaktionsbedingungen:

| | |
|---|---|
| Reaktionsvolumen (Batch): | 500 ml |
| Katalysatormenge: | 10,1 g/l |
| Substratanfangskonzentration: | 0,4 mol/l |
| pH: | 9,0 |
| Temperatur: | 60 °C |
| Druck: | Normaldruck |
| O₂-Flussrate: | 0,5 l/min |
| Titrationsmittel: | NaOH (16 Gew.-% Trockensubstanz (TS) auf Gesamtmasse) |
| Rührgeschwindigkeit: | 300 upm (Rührwerk) |

Die Reaktionsdauer betrug einmal 5,8 h (Beispiel 1a) und einmal 23,1 h (Beispiel 1b) bei ansonsten gleichen Reaktionsbedingungen.

### Beispiel 2 - erfindungsgemäßes zweistufiges Verfahren

Als Katalysator wurde der gleiche Katalysator verwendet wie in Beispiel 1 (3,5 %Pt/1 % Bi/C (=Norit SX UltraCat)). Die HMF-Oxidation erfolgte unter folgenden Reaktionsbedingungen:

| | |
|---|---|
| Reaktionsvolumen: | 500 ml |
| Katalysatormenge: | 9,5 g/l |
| Substratanfangskonzentration: | 0,4 mol/l |
| pH, Schritte a) und b): | 9,0 |
| pH, Schritt c) | 12,0 |
| Temperatur: | 60 °C |
| Druck, Schritte b) und c): | Normaldruck |
| O₂-Flussrate, Schritte b) und c): | 0,5 l/min |
| Titrationsmittel, Schritte b) und c): | NaOH (16 Gew.-% Trockensubstanz (TS) auf Gesamtmasse) |
| Rührgeschwindigkeit, Schritte b) und c): | 300 upm (Rührwerk) |

Die Umstellung auf Verfahrensschritt c) erfolgte nach Zugabe von 1,13 Äquivalenten NaOH in Verfahrensschritt b), im Rahmen dessen der pH-Wert konstant gehalten wurde.

Zum Einstellen und Aufrechterhalten des pH-Wertes in Verfahrensschritt c) wurden 0,88 Äquivalente NaOH zugegeben.

Die Ergebnisse, die unter den Reaktionsbedingungen aus Beispiel 1 und 2 erhalten wurden, sind in Tabelle 1 dargestellt.

**Tabelle 1: Gegenüberstellung der Ergebnisse aus Beispiel 1 und 2.**

| Verfahren | Beispiel 1a - einstufiges Vergleichsverfahren | Beispiel 1b - einstufiges Vergleichsverfahren | Beispiel 2 - erfindungsgemäß 2-stufig |
|---|---|---|---|
| Reaktionszeit | 5,8 h | 23,1 h | 0,8 h |
| C-Bilanz am Reaktionsende | 85,6 | 87,7 | 92,3 |
| FDCA [g/100 g] | 2,19 | 4,65 | 4,62 |
| FFCA [g/100 g] | 1,96 | 0,4 | 0,08 |
| HFCA [g/100 g] | 0,06 | 0,02 | 0,02 |

Es ist zu erkennen, dass im erfindungsgemäßen Beispiel 2 die C-Bilanz deutlich besser ist als im Vergleichsbeispiel 1. Ferner konnte in Beispiel 2 eine vergleichbare Menge FDCA in erheblich kürzerer Zeit hergestellt werden als in Beispiel 2b, wobei der Gehalt an Nebenkomponenten geringer ausfällt.

### Beispiel 3 - Zugabe von 2 und 4 Laugenäquivalenten zu Reaktionsbeginn (Vergleichsbeispiel)

Als Katalysator wurde ein Hydrotalcit geträgerter Pt/Bismuth-Katalysator (2,5 % Pt/1 % Bi/HT) eingesetzt. Die HMF-Oxidation erfolgte unter folgenden Reaktionsbedingungen:

| | |
|---|---|
| Reaktionsvolumen (Batch): | 500 ml |
| Katalysatormenge: | 5,0 g/l |
| Substratanfangskonzentration: | 0,4 mol/l |
| pH: | kein konstanter pH-Wert, pH Start 13,2 - pH Ende 9,6 |
| Temperatur: | 60 °C |
| Druck: | Normaldruck |
| O₂-Flussrate: | 0,5 l/min |
| Titrationsmittel: | NaOH (16 Gew.-% Trockensubstanz (TS) auf Gesamtmasse) |
| Rührgeschwindigkeit: | 300 upm (Rührwerk) |

### Beispiel 3a:

Die gesamte benötigte NaOH-Menge an 100 g NaOH (16 wt%) entsprechend 2 Äquivalenten NaOH pro mol HMF wurde direkt zu Reaktionsbeginn zugesetzt.

### Beispiel 3b:

Die gesamte benötigte NaOH-Menge an 200 g NaOH (16 wt%) entsprechend 4 Äquivalenten NaOH pro mol HMF wurde direkt zu Reaktionsbeginn zugesetzt.

### Beispiel 4 - erfindungsgemäßes Verfahren

Es wurde der gleiche Katalysator wie in Beispiel 3 eingesetzt. Die HMF-Oxidation erfolgte unter folgenden Reaktionsbedingungen:

| | |
|---|---|
| Reaktionsvolumen (Batch): | 500 ml |
| Katalysatormenge: | 5,0 g/l |
| Substratanfangskonzentration: | 0,4 mol/l |
| pH, Schritte a) und b): | 9,0 |
| pH, Schritt c): | 11,6 |
| Temperatur, Schritte b) und c): | 60 °C |
| Druck, Schritte b) und c): | Normaldruck |
| O₂-Flussrate, Schritte b) und c): | 0,5 l/min |
| Titrationsmittel, Schritte b) und c): | NaOH (16 Gew.-% Trockensubstanz (TS) auf Gesamtmasse) |
| Rührgeschwindigkeit, Schritte b) und c): | 300 upm (Rührwerk) |

Die Umstellung auf Verfahrensschritt c) erfolgte nach Zugabe von 0,8 Äquivalenten NaOH in Verfahrensschritt b), im Rahmen dessen der pH-Wert konstant gehalten wurde. Zum Einstellen und Aufrechterhalten des pH-Wertes in Verfahrensschritt c) wurden 1,2 Äquivalente NaOH zugegeben. Insgesamt wurden 2 NaOH-Äquivalente zugesetzt.

Die Ergebnisse aus den Beispielen 3a, 3b und 4 sind in Tabelle 2 dargestellt.

**Tabelle 2: Gegenüberstellung der Versuchsergebnisse aus Beispiel 3 und 4.**

| Verfahren | Beispiel 3a - 2 Äquivalente NaOH Direktzugabe | Beispiel 3b - 4 Äquivalente NaOH Direktzugabe | Beispiel 4 - erfindungsgemäß 2-stufig |
|---|---|---|---|
| Reaktionszeit | 5 h | 5 h | 4,82 h |
| C-Bilanz am Reaktionsende | 11,31 | 21,07 | 95,14 |
| FDCA [g/100 g] | 0,00 | 0,84 | 4,81 |
| FFCA [g/100 g] | 0,29 | 0,01 | 0,06 |
| HFCA [g/100 g] | 0,26 | 0,11 | 0,00 |

Es ist zu erkennen, dass im erfindungsgemäßen Beispiel 4 eine deutlich bessere C-Bilanz, mehr FDCA und weniger Zwischenprodukte erhalten wurde als in den beiden Vergleichsbeispielen 3a und 3b.

### Beispiel 5 - Variation des pH-Wertes in Verfahrensschritt b):

Als Katalysator wurde ein Hydrotalcit geträgerter Platin/Bi-Katalysator (2,5 % Pt/1 % Bi/HT) eingesetzt.

Die HMF-Oxidation erfolgte unter folgenden Reaktionsbedingungen in Verfahrensschritten a) und b):

| | |
|---|---|
| Reaktionsvolumen (Batch): | 500 ml |
| Katalysatormenge: | 5,0 g/l |
| Substratanfangskonzentration: | 0,4 mol/l |
| Temperatur: | 60 °C |
| Druck: | Normaldruck |
| O₂-Flussrate: | 0,5 l/min |
| Titrationsmittel: | NaOH 16 (16 Gew.-% Trockensubstanz (TS) auf Gesamtmasse) |
| Rührgeschwindigkeit: | 900 upm (Büchi-Begasungsrührer) |

Die pH-Werte in Verfahrensschritt b) wurden wie folgt gewählt (Tabelle 3) und durch Zugabe von entsprechenden Mengen an NaOH konstant gehalten. Verfahrensschritt c) wurde nicht durchgeführt.

**Tabelle 3: pH-Werte in Verfahrensschritt b) für die Versuche 5a-f.**

| Nr. | pH-Wert, Schritt b) | In Schritt b) zugesetzte Äquivalente NaOH |
|---|---|---|
| 5a | 7,0 | 0,6 |
| 5b | 8,0 | 1,0 |
| 5c | 9,0 | 1,3 |
| 5d | 10,0 | 1,6 |
| 5e | 11,0 | 1,9 |
| 5f | 12,0 | 2,1 |

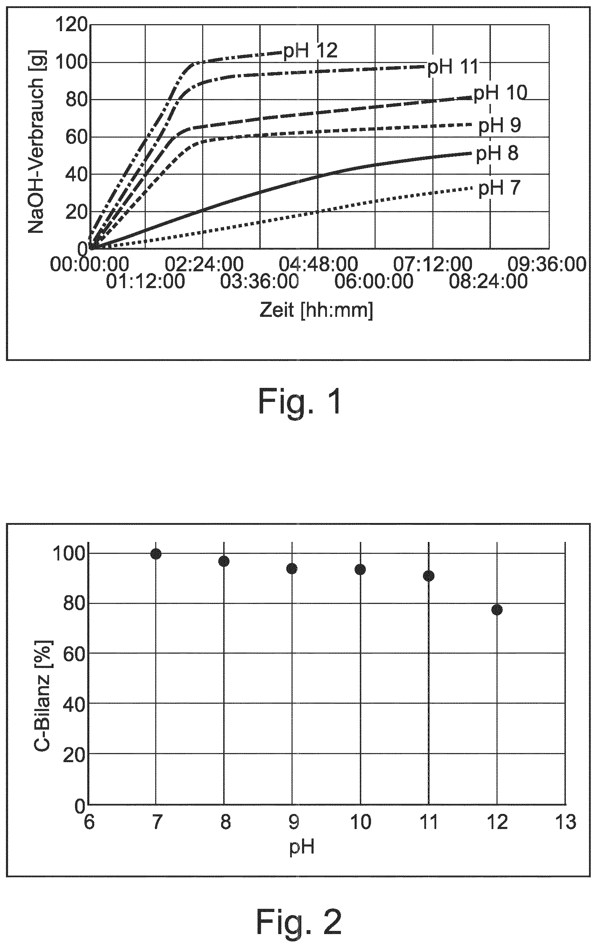

In Figur 1 ist ein Vergleich der Titrationskurven gezeigt.

Es ist klar ersichtlich, dass die Geschwindigkeit der Umsetzung in Verfahrensschritt b) abhängig vom pH-Wert ist. Mit steigendem pH-Wert wurde die Reaktion schneller. In Figur 2 ist die C-Bilanz am Endpunkt des Verfahrensschritts b) (Zugabe von 1 Äquivalent NaOH (entspricht 50 g NaOH) gezeigt.

Die höhere Reaktionsgeschwindigkeit bei höheren pH-Wert geht mit zunehmendem Verlust an Produkt und/oder Zwischenprodukten einher. Akzeptable C-Bilanzen von ≥ 93 % werden nur für pH-Werte ≤ pH 10 erreicht.

### Beispiel 6 - Variation des pH-Wertes in Verfahrensschritt c):

Als Katalysator wurde ein Hydrotalcit geträgerter Platin/Bi-Katalysator (2,5 % Pt/1 % Bi/HT) eingesetzt.

Die HMF-Oxidation erfolgte unter folgenden Reaktionsbedingungen:

| | |
|---|---|
| Reaktionsvolumen (Batch): | 500 ml |
| Katalysatormenge: | 5,0 g/l |
| Substratanfangskonzentration: | 0,4 mol/l |
| pH-Wert, Schritte a) und b): | 9,0 |
| Temperatur, Schritte b) und c): | 60 °C |
| Druck, Schritte b) und c): | Normaldruck |
| O₂-Flussrate, Schritte b) und c): | 0,5 l/min |
| Rührgeschwindigkeit, Schritte b) und c): | 900 upm (Büchi-Begasungsrührer) |

Die Umstellung auf Verfahrensschritt c) erfolgte nach Zugabe von 0,8 Äquivalenten NaOH in Verfahrensschritt b).

Die pH-Werte für Verfahrensschritt c) wurde für die unterschiedlichen Beispiele so gewählt wie in Tabelle 4 angegeben und durch Zugabe von entsprechenden Mengen an NaOH eingestellt und konstant gehalten:

**Tabelle 4: pH-Werte in Verfahrensschritt c) für die Versuche 6a - d.**

| Nr. | pH-Wert, Schritt c) | In Schritt c) zugesetzte Äquivalente NaOH |
|---|---|---|
| 6a | 10 | 0,8 |
| 6b | 11 | 1,2 |
| 6c | 12 | 1,2 |
| 6d | 13 | 1,6 |

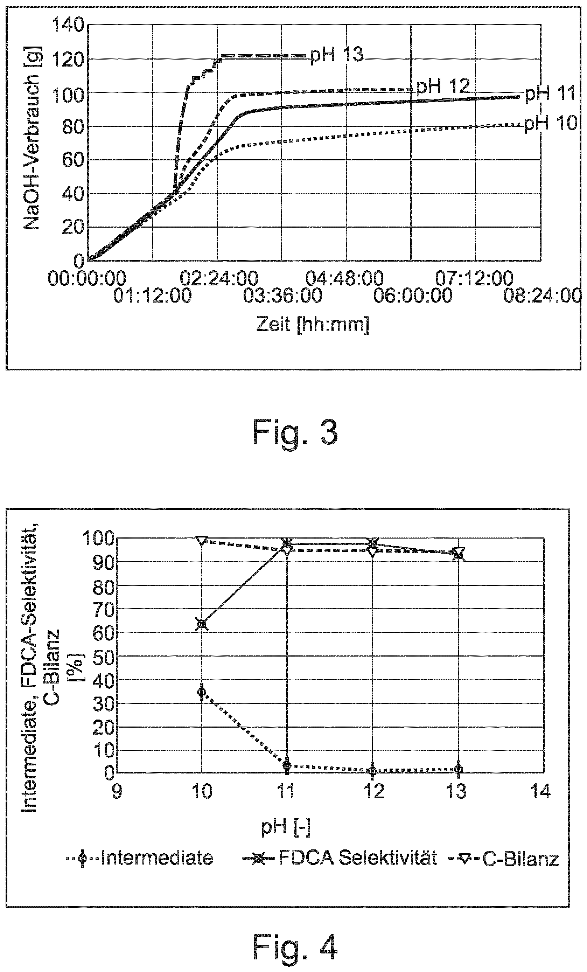

In Figur 3 sind die Titrationskurven für die verschiedenen pH-Werte des Verfahrensschrittes c) gezeigt.

In Figur 4 sind die C-Bilanz, die FDCA-Selektivität und die Selektivität für die Intermediate in Abhängigkeit des pH-Werts dargestellt.

Die C-Bilanz ist mit 98 % für pH 10 am höchsten, die Selektivität für FDCA jedoch mit 64 % auch am geringsten. Die hohe Selektivität für die Intermediate (34 %) zeigt, dass die Reaktion noch nicht beendet war. Die höchste Selektivität für FDCA zeigt sich mit 97,5 % bei einem pH von 12. Unter diesen Bedingungen ist auch die Selektivität für die Intermediate mit 0,44 % am geringsten.

### Beispiel 7 - Einsatz unterschiedlicher Katalysatoren für Stufe 1 und 2

Katalysator 1. Stufe: 1 % Au/Al₂O₃
Katalysator 2. Stufe: 2,5 % Pt/ 1,0 % Bi/HAT (HT = Hydrotalcit)
Die Reaktion erfolgte unter folgenden Reaktionsbedingungen:
Reaktionsvolumen: 500 ml
Katalysatormenge 1. Stufe: 12,5 g/l
Katalysatormenge 2. Stufe: 5,0 g/l
Substratanfangskonzentration: 0,4 mol/l
pH 1. Stufe: 9,0
pH 2. Stufe: 11,7
Temperatur: 60 °C
Druck: Normaldruck
O2-Flussrate: 500 ml/min
Rührgeschwindigkeit: 900 rpm (Büchi-Begasungsrührer)

Nach Zugabe von 0,8 Äquivalenten NaOH in Verfahrensschritt b) wird der Katalysator mittels Filtration abgetrennt und die filtrierte Lösung ohne weitere Behandlung in der zweiten Stufe in Verfahrensschritt c) unter Zugabe von 1,1 Äquivalente NaOH eingesetzt.

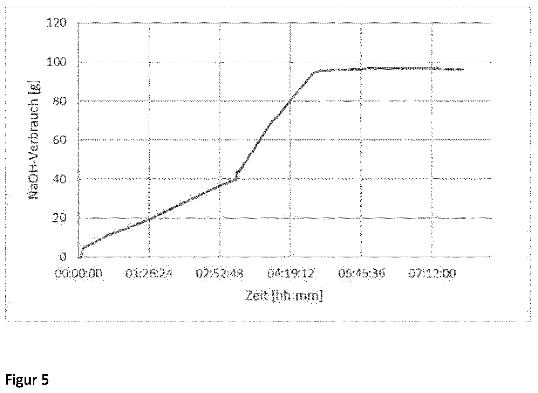

Figur 5 zeigt die NaOH-Verbrauchskurve bei der erfindungsgemäßen Verfahrensweise unter Einsatz zweier unterschiedlicher Katalysatoren.

**Tabelle 5 zeigt die Ergebnisse.**

| | |
|---|---|
| HMF-Umsatz | 100 % |
| C-Bilanz | 97,03 % |
| FDCA-Selektivität | 96,83 % |
| Intermediate | 0,21 % |

Es wird ein vollständiger HMF-Umsatz bei akzeptabler C-Bilanz und hoher FDCA-Selektivität erreicht.

## Patentansprüche

1. Verfahren zur Herstellung von 2,5-Furandicarboxylsäure (FDCA) aus Hydroxymethylfurfural (HMF) durch 2-stufige Oxidation des HMF, umfassend die folgenden Verfahrensschritte:
a) Bereitstellen einer wässrigen Lösung enthaltend HMF mit einem pH-Wert in einem Bereich von 7,0 bis 10,0, mindestens einer Lauge und mindestens eines Edelmetallkatalysators,
b) Umsetzen der wässrigen Lösung enthaltend HMF in Anwesenheit mindestens eines gemäß Verfahrensschritt a) bereitgestellten Edelmetallkatalysators unter oxidativen Bedingungen und unter Aufrechterhalten eines konstanten pH-Wertes, der in einem Bereich von 7,0 bis 10,0 liegt, durch Zugabe von 0,7 bis 1,3 Stoffmengenäquivalenten (eq) (bezogen auf Stoffmenge des HMF in der eingesetzten wässrigen Lösung) der mindestens einen Lauge zum Erhalt einer Zwischenproduktlösung enthaltend Hydroxymethyl-2-furancarboxylsäure (HFCA) und/oder 5-Formyl-2-furancarboxylsäure (FFCA) und
c) Umsetzen der Zwischenproduktlösung in einem pH-Wert-Bereich von 10,5 bis 14,0 in Anwesenheit mindestens eines gemäß Verfahrensschritt a) bereitgestellten Edelmetallkatalysators unter oxidativen Bedingungen zum Erhalt einer Lösung enthaltend 2,5-Furandicarboxylsäure (FDCA).

2. Verfahren nach Anspruch 1, wobei Verfahrensschritt c) unter Einstellen und Aufrechterhalten eines konstanten pH-Wertes, der in dem Bereich von 10,5 bis 14,0 liegt, durch Zugabe der mindestens einen Lauge durchgeführt wird.

3. Verfahren nach Anspruch 1 oder 2, wobei in einem Verfahrensschritt a0) vor Verfahrensschritt a) der pH-Wert einer wässrigen Lösung enthaltend HMF mit einem pH-Wert von 3,0 bis 6,0 auf einen pH-Wert in einem Bereich von 7,0 bis 10,0 erhöht wird, insbesondere unter nicht oxidativen Bedingungen, insbesondere ohne Zufuhr von Luft und Sauerstoff.

4. Verfahren nach einem der vorhergehenden Ansprüche, wobei in Verfahrensschritt a) mindestens zwei unterschiedliche Laugen bereitgestellt werden und in Verfahrensschritt b) eine erste Lauge, insbesondere eine schwache Lauge, und in Verfahrensschritt c) eine zweite Lauge, insbesondere eine starke Lauge, verwendet werden.

5. Verfahren nach einem der vorhergehenden Ansprüche, wobei die mindestens eine, insbesondere starke, Lauge NaOH oder KOH ist.

6. Verfahren nach einem der vorhergehenden Ansprüche, wobei in Verfahrensschritt c) 0,7 bis 1,3 Stoffmengenäquivalente (eq) der mindestens einen Lauge (bezogen auf Stoffmenge des HMF in der eingesetzten wässrigen Lösung) zugegeben werden.

7. Verfahren nach einem der vorhergehenden Ansprüche, wobei die oxidativen Bedingungen, insbesondere während der Verfahrensschritte b) und/oder c), Reaktionsbedingungen sind, die zu einer Zufuhr von Sauerstoff oder Luft in die wässrige Lösung führen, insbesondere durch eine Luft- oder Sauerstoff-Begasungsvorrichtung, insbesondere einen Begasungsrührer, oder ein Gebläse oder/und einen Strömungskanal, insbesondere unter gleichzeitiger mechanischer Agitation der Lösung, zum Beispiel durch ein Rührwerk oder eine Verwirbelungseinrichtung.

8. Verfahren nach einem der vorhergehenden Ansprüche, wobei die Verfahrensschritte b) und c) unter mechanischer Agitation durchgeführt werden, insbesondere unter Rühren mittels eines Rührwerks oder einer Verwirbelungseinrichtung.

9. Verfahren nach einem der vorhergehenden Ansprüche, wobei der mindestens eine Edelmetallkatalysator mindestens ein Edelmetall aufweist, ausgewählt aus der Gruppe bestehend aus Kupfer, Ruthenium, Rhodium, Kobalt, Iridium, Platin, Palladium, Gold und Silber.

10. Verfahren nach einem der vorhergehenden Ansprüche, wobei der mindestens eine Edelmetallkatalysator mindestens ein Edelmetall und mindestens ein Dotiermittel, insbesondere Blei, Zinn, Thallium, Tellur, Kobalt, oder Bismut, aufweist, insbesondere ein Pt-Bi-Edelmetallkatalysator ist.

11. Verfahren nach einem der vorhergehenden Ansprüche, wobei Verfahrensschritt b), c) oder beide bei einer Temperatur von 40 bis 100 °C durchgeführt wird.

12. Verfahren nach einem der vorhergehenden Ansprüche, wobei im Anschluss an Verfahrensschritt c) in einem Verfahrensschritt d) der mindestens eine Edelmetallkatalysator aus der Lösung enthaltend FDCA abgetrennt wird, insbesondere mittels Filtration.

13. Verfahren nach einem der vorhergehenden Ansprüche, wobei im Anschluss an Verfahrensschritt c) oder d) in einem Verfahrensschritt f) das in Verfahrensschritt c) oder d) erhaltene FDCA protoniert wird, insbesondere, indem die Lösung enthaltend FDCA auf einen pH-Wert von 1,0 bis 6,5 eingestellt und protoniertes FDCA erhalten wird.

14. Verfahren nach einem der vorhergehenden Ansprüche, wobei im Anschluss an Verfahrensschritt c) oder d) in einem Verfahrensschritt e) die Lösung enthaltend das FDCA aufgereinigt wird.

15. Verfahren nach einem der vorhergehenden Ansprüche, wobei im Anschluss an Verfahrensschritt c), d) oder f) in einem Verfahrensschritt g) das protonierte oder nichtprotonierte FDCA aufgereinigt wird, bevorzugt mittels Waschung, Umkristallisation, Schmelzen, Adsorption oder einer Kombination davon.

16. Verfahren nach einem der vorhergehenden Ansprüche, wobei im Anschluss an Verfahrensschritt c), d), e), f) oder g) in einem Verfahrensschritt h) protoniertes oder nicht-protoniertes FDCA in einen Methyl-, Ethyl- oder Dimethylester überführt wird.

17. Verfahren zur Herstellung von Polyestern, Polyamiden oder Polyurethanen, insbesondere Polyethylenfuranoat (PEF), wobei ein Verfahren nach einem der Ansprüche 1 bis 16 und anschließend ein Polymerisieren des aus Verfahrensschritt c), d), e), f) oder g) erhaltenen FDCA durchgeführt wird.

## Claims

1. A process for the preparation of 2,5-furandicarboxylic acid (FDCA) from hydroxymethylfurfural (HMF) by 2-stage oxidation of the HMF, comprising the following process steps:
a) providing an aqueous solution containing HMF having a pH value in a region of 7.0 to 10.0, at least one lye and at least one precious metal catalyst,
b) reacting the aqueous solution containing HMF in the presence of at least one precious metal catalyst provided according to process step a) under oxidative conditions and while maintaining a constant pH value, which is in a region of 7.0 to 10.0, by adding 0.7 to 1.3 amount of substance equivalents (eq) (based on the amount of substance of the HMF in the aqueous solution used) of the at least one lye to obtain an intermediate product solution containing hydroxymethyl-2-furancarboxylic acid (HFCA) and/or 5-formyl-2-furancarboxylic acid (FFCA) and
c) reacting the intermediate product solution in a pH value region of 10.5 to 14.0 in the presence of at least one precious metal catalyst provided according to process step a) under oxidative conditions to obtain a solution containing 2,5-furandicarboxylic acid (FDCA).

2. The process of claim 1, wherein process step c) is carried out while adjusting and maintaining a constant pH value, which is in the region of 10.5 to 14.0, by adding the at least one lye.

3. The process of claim 1 or 2, wherein in a process step a0) prior to process step a) the pH value of an aqueous solution containing HMF with a pH value of 3.0 to 6.0 is increased to a pH value in a region of 7.0 to 10.0, in particular under non-oxidative conditions, in particular without the addition of air and oxygen.

4. The process of one of the preceding claims, wherein in process step a) at least two different lyes are provided and in process step b) a first lye, in particular a weak lye, and in process step c) a second lye, in particular a strong lye, are used.

5. The process of one of the preceding claims, wherein the at least one, in particular strong, lye is NaOH or KOH.

6. The process of one of the preceding claims, wherein in process step c) 0.7 to 1.3 amount of substance equivalents (eq) of the at least one lye (based on the amount of substance of the HMF in the aqueous solution used) are added.

7. The process of one of the preceding claims, wherein the oxidative conditions, in particular during the process steps b) and/or c), are reaction conditions which lead to a supply of oxygen or air into the aqueous solution, in particular by an air or oxygen gassing device, in particular a gassing stirrer or a blower or/and a flow channel, in particular with simultaneous mechanical agitation of the solution, for example by an agitator or a turbulence means.

8. The process of one of the preceding claims, wherein the process steps b) and c) are carried out with mechanical agitation, in particular with stirring by means of an agitator or a turbulence means.

9. The process of one of the preceding claims, wherein the at least one precious metal catalyst has at least one precious metal selected from the group consisting of copper, ruthenium, rhodium, cobalt, iridium, platinum, palladium, gold and silver.

10. The process of one of the preceding claims, wherein the at least one precious metal catalyst has at least one precious metal and at least one dopant, in particular lead, tin, thallium, tellurium, cobalt, or bismuth, in particular is a Pt-Bi precious metal catalyst.

11. The process of one of the preceding claims, wherein process step b), c) or both is carried out at a temperature of 40 to 100 °C.

12. The process of one of the preceding claims, wherein, following process step c), in a process step d) the at least one precious metal catalyst is separated from the solution containing FDCA, in particular by means of filtration.

13. The process of one of the preceding claims, wherein, following process step c) or d), in a process step f) the FDCA obtained in process step c) or d) is protonated, in particular by adjusting the solution containing FDCA to a pH value of 1.0 to 6.5 and obtaining protonated FDCA.

14. The process of one of the preceding claims, wherein, following process step c) or d), in a process step e) the solution containing the FDCA is purified.

15. The process of one of the preceding claims, wherein, following process step c), d) or f), in a process step g) the protonated or non-protonated FDCA is purified, preferably by washing, recrystallisation, melting, adsorption or a combination thereof.

16. The process of one of the preceding claims, wherein, following process step c), d), e), f) or g), in a process step h) protonated or non-protonated FDCA is converted into a methyl, ethyl or dimethyl ester.

17. A process for the preparation of polyesters, polyamides or polyurethanes, in particular polyethylene furanoate (PEF), wherein a process of one of claims 1 to 16 and subsequently a polymerising of the FDCA obtained from process step c), d), e), f) or g) is carried out.

## Revendications

1. Procédé de préparation d'acide 2,5-furanedicarboxylique (FDCA) à partir d'hydroxyméthylfurfural (HMF) par oxydation en 2 étages du HMF, comprenant les étapes de procédé suivantes :
a) mise à disposition d'une solution aqueuse contenant du HMF présentant une valeur de pH dans une plage de 7,0 à 10,0, au moins une solution alcaline et au moins un catalyseur de métal noble,
b) transformation de la solution aqueuse contenant du HMF en présence d'au moins un catalyseur de métal noble mis à disposition selon l'étape de procédé a) dans des conditions oxydantes et tout en maintenant une valeur de pH constante, qui se situe dans la plage de 7,0 à 10,0, par ajout de 0,7 à 1,3 équivalent de quantité de matière (éq) (par rapport à la quantité de matière du HMF dans la solution aqueuse utilisée) de ladite au moins une solution alcaline afin d'obtenir une solution de produit intermédiaire contenant de l'acide hydroxyméthyl-2-furanecarboxylique (HFCA) et/ou de l'acide 5-formyl-2-furanecarboxylique (FFCA) et
c) transformation de la solution de produit intermédiaire dans une plage de valeur de pH de 10,5 à 14,0 en présence d'au moins un catalyseur de métal noble mis à disposition selon l'étape de procédé a) dans des conditions oxydantes afin d'obtenir une solution contenant de l'acide 2,5-furanedicarboxylique (FDCA).

2. Procédé selon la revendication 1, dans lequel l'étape de procédé c) est réalisée tout en réglant et en maintenant une valeur de pH constante qui se situe dans la plage de 10,5 à 14,0 par ajout de ladite au moins une solution alcaline.

3. Procédé selon la revendication 1 ou 2, dans lequel, dans une étape de procédé a0) avant l'étape de procédé a), la valeur de pH d'une solution aqueuse contenant du HMF présentant une valeur de pH de 3,0 à 6,0 est augmentée à une valeur de pH dans une plage de 7,0 à 10,0, en particulier dans des conditions non oxydantes, en particulier sans introduction d'air et d'oxygène.

4. Procédé selon l'une des revendications précédentes, dans lequel, dans l'étape de procédé a), au moins deux lessives alcalines différentes sont mises à disposition et, dans l'étape de procédé b), une première solution alcaline, en particulier une solution faiblement alcaline est utilisée et, dans l'étape de procédé c), une deuxième solution alcaline, en particulier une solution alcaline forte, est utilisée.

5. Procédé selon l'une des revendications précédentes, dans lequel ladite au moins une solution alcaline, en particulier forte, est le NaOH ou le KOH.

6. Procédé selon l'une des revendications précédentes, dans lequel, dans l'étape de procédé c), 0,7 à 1,3 équivalent de quantité de matière (éq) de ladite au moins une solution alcaline (par rapport à la quantité de matière du HMF dans la solution aqueuse utilisée) est ajouté.

7. Procédé selon l'une des revendications précédentes, dans lequel les conditions oxydantes, en particulier pendant les étapes de procédé b) et/ou c), sont des conditions de réaction qui entraînent une introduction d'oxygène ou d'air dans la solution aqueuse, en particulier par un dispositif d'injection d'air ou d'oxygène, en particulier un agitateur à injection ou un ventilateur et/ou un canal d'écoulement, en particulier avec une agitation mécanique simultanée de la solution, par exemple par un agitateur ou un dispositif de tourbillonnement.

8. Procédé selon l'une des revendications précédentes, dans lequel les étapes de procédé b) et c) sont réalisées sous agitation mécanique, en particulier sous agitation au moyen d'un agitateur ou d'un dispositif de tourbillonnement.

9. Procédé selon l'une des revendications précédentes, dans lequel ledit au moins un catalyseur de métal noble présente au moins un métal noble choisi dans le groupe constitué par le cuivre, le ruthénium, le rhodium, le cobalt, l'iridium, le platine, le palladium, l'or et l'argent.

10. Procédé selon l'une des revendications précédentes, dans lequel ledit au moins un catalyseur de métal noble présente au moins un métal noble et au moins un agent de dopage, en particulier du plomb, de l'étain, du thallium, du tellure, du cobalt ou du bismuth, et est en particulier un catalyseur de métal noble-Pt-Bi.

11. Procédé selon l'une des revendications précédentes, dans lequel l'étape de procédé b), c) ou les deux étapes est/sont réalisée(s) à une température de 40 à 100°C.

12. Procédé selon l'une des revendications précédentes, dans lequel, après l'étape de procédé c), dans une étape de procédé d), ledit au moins un catalyseur de métal noble est séparé de la solution contenant du FDCA, en particulier par filtration.

13. Procédé selon l'une des revendications précédentes, dans lequel, après l'étape de procédé c) ou d), dans une étape de procédé f), le FDCA obtenu dans l'étape de procédé c) ou d) est protoné, en particulier lorsque la solution contenant du FDCA est réglée à une valeur de pH de 1,0 à 6,5 et du FDCA protoné est obtenu.

14. Procédé selon l'une des revendications précédentes, dans lequel, après l'étape de procédé c) ou d), dans une étape de procédé e), la solution contenant le FDCA est purifiée.

15. Procédé selon l'une des revendications précédentes, dans lequel, après l'étape de procédé c), d) ou f), dans une étape de procédé g), le FDCA protoné ou non protoné est purifié, de préférence par lavage, recristallisation, fusion, adsorption ou une combinaison correspondante.

16. Procédé selon l'une des revendications précédentes, dans lequel, après l'étape de procédé c), d), e), f) ou g), dans une étape de procédé h), le FDCA protoné ou non protoné est transformé en un ester méthylique, éthylique ou diméthylique.

17. Procédé de préparation de polyesters, de polyamides ou de polyuréthanes, en particulier de polyéthylène furanoate (PEF), dans lequel un procédé selon l'une des revendications 1 à 16 et ensuite une polymérisation du FDCA obtenu de l'étape de procédé c), d), e), f) ou g) sont réalisés.
